(19) Europäisches Patentamt
European Patent Office
Office européen des brevets

(11) **EP 3 169 274 B1**

(12) **EUROPÄISCHE PATENTSCHRIFT**

(45) Veröffentlichungstag und Bekanntmachung des Hinweises auf die Patenterteilung:
**30.10.2024 Patentblatt 2024/44**

(21) Anmeldenummer: **15742203.1**

(22) Anmeldetag: **15.07.2015**

(51) Internationale Patentklassifikation (IPC):
**A61F 2/07** (2013.01) **A61F 2/06** (2013.01)

(52) Gemeinsame Patentklassifikation (CPC):
**A61F 2/07;** A61F 2002/061; A61F 2240/00; A61F 2250/0024

(86) Internationale Anmeldenummer:
**PCT/EP2015/066200**

(87) Internationale Veröffentlichungsnummer:
**WO 2016/008944 (21.01.2016 Gazette 2016/03)**

(54) **GEFÄSSPROTHESENSYSTEM UND HERSTELLUNGSVERFAHREN DES GEFÄSSPROTHESENSYSTEMS**

VASCULAR PROSTHESIS SYSTEM AND PRODUCTION METHOD OF THE VASCULAR PROSTHESIS

SYSTÈME DE PROTHÈSE VASCULAIRE ET PROCÉDÉ DE FABRICATION DE LA PROTHÈSE VASCULAIRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.07.2014 DE 102014110013**

(43) Veröffentlichungstag der Anmeldung:
**24.05.2017 Patentblatt 2017/21**

(73) Patentinhaber: **JOTEC GmbH
72379 Hechingen (DE)**

(72) Erfinder: **BRAUN, Michael
71522 Backnang (DE)**

(74) Vertreter: **Witte, Weller & Partner Patentanwälte mbB
Postfach 10 54 62
70047 Stuttgart (DE)**

(56) Entgegenhaltungen:
EP-A1- 2 821 030          EP-B1- 0 880 948
WO-A1-00/53119          WO-A1-03/006097
WO-A1-2010/024880          WO-A2-2006/111801
DE-A1- 102012 103 987          US-A1- 2005 240 261
US-A1- 2009 157 164          US-A1- 2012 046 728
US-A1- 2013 296 998          US-A1- 2014 018 619

**Beschreibung**

Die vorliegende Erfindung betrifft ein Gefäßprothesensystem mit einer Gefäßprothese und ein Herstellungsverfahren dafür.

Bei Defekten an den Hauptgefäßen des menschlichen Körpers, insbesondere der Aorta, ist es bekannt, Gefäßprothesen, die auch als Graft, Endografts oder Stentgrafts bezeichnet werden, in das Blutgefäß einzusetzen. Solche Defekte sind beispielsweise so genannte Aneurysmen. Grafts weisen hierbei in der Regel, zumindest im eingebrachten Zustand eine zylindrische Form auf. Ein Problem, das bei dieser Behandlung des betroffenen Blutgefäßes besteht, ist, dass in der Regel von den Hauptgefä-ßen mehrere Gefäße abzweigen. Befindet sich der Defekt in der Nähe einer solchen Abzweigung, so ist das zuverlässige Unterstützen der Gefäßwand ohne gleichzeitiges Blockieren der Abzweigung mit den herkömmlichen zylindrischen Grafts nicht möglich.

Aus diesem Grund sind Gefäßprothesen vorgeschlagen worden, bei denen vor dem Einbringen in das Blutgefäß ein Durchlass ausgeschnitten wird, durch den eine Gefäßstütze in die Gefäßprothese eingebracht und mit dieser verbunden werden kann. Ein Nachteil bei diesen so genannten fenestrierten Gefäßprothesen ist, dass zum einen die Position der Abzweigung relativ zu der Position der Gefäßprothese vor dem Einbringen der Gefäßprothese genau bestimmt werden muss. Zum anderen ist das Positionieren der Gefäßprothese schwierig, da der Durchlass im eingebrachten Zustand exakt mit der Abzweigung ausgerichtet sein muss.

Aus der DE 10 2012 103 987 A1 ist eine Gefäßprothese mit einem Fenestrierungsbereich bekannt. Ferner offenbaren die US 2013/0296998 A1 und die US 2012/0046728 A1 Gefäßprothesen mit Fenestrierungen.

Zudem sind Gefäßstützen vorgeschlagen, bei denen über einen Teil der Länge der Gefäßprothese die Maschenweite des Materials der Gefäßprothese größer gewählt ist. Dieser Bereich kann dann mit der Abzweigung ausgerichtet werden und durch einen Ballon kann ein Durchlass in dem Material der Gefäßprothese erzeugt werden. Die größere Maschenweite dient hierbei lediglich der einfacheren Ausdehnung mittels des Ballons. Das Einführen des Drahtes auf dem der Ballon gehalten wird, ist aber weiterhin schwierig, da die Maschenweite nicht zu groß gewählt werden darf um ein ungewolltes Austreten von Blut aus der Gefäßprothese und Vorbeiströmen an der Gefäßprothese verhindert werden muss.

Der Erfindung liegt somit die Aufgabe zugrunde eine Lösung zu schaffen, die ein einfaches Herstellen einer Gefäßprothese und Einbringen der Gefäßprothese in ein Hauptblutgefäß und zuverlässiges Abdichten der Prothesenrandzonen, insbesondere im Bereich der Prothesenabzweigungen auf einfache Weise erlaubt.

Gemäß einem ersten Aspekt der Erfindung wird die Aufgabe gelöst durch ein Gefäßprothesensystem, umfassend zumindest eine Gefäßprothese zum Einführen in ein Blutgefäß eines menschlichen Körpers, wobei die Gefäßprothese ein proximales Ende, ein distales Ende, sowie einen sich zwischen dem proximalen und dem distalen Ende erstreckenden länglichen hohlzylindrischen Grundkörper mit einer Längsachse c und einem Umfang b aufweist, welcher ein Gefäßprothesenmaterial zur Ausbildung eines umfänglich im Wesentlichen geschlossenen Mantels aufweist, wobei das Gefäßprothesenmaterial eine Gewebestruktur aus miteinander verwobenen Fäden und mit einer Maschendichte besitzt; bei dem erfindungsgemäßen Gefäßprothesensystem weist ferner die Gefäßprothese im Gefäßprothesenmaterial zumindest einen, zwischen dem proximalen und dem distalen Ende gelegenen Lochungsbereich mit mehreren Löchern auf, die einen Durchmesser x aufweisen, der ausgewählt ist aus einem Bereich von 1,0 mm bis 1,3 mm, und wobei die mehreren Löcher einen geschlossenen umlaufenden Rand aufweisen, über welchen Rand sich kein Prothesenmaterial in Richtung der Löcher über den Rand hinaus erstreckt.

Die der Erfindung zugrunde liegende Aufgabe wird auf diese Weise vollkommen gelöst: Die definierten Durchlässe in der Gefäßprothese dienen zum einen als Durchlass zur Aufnahme einer Gefäßstütze, wodurch eine ausreichende Blutzufuhr zu Abzweigungsgefäßen gewährleistet wird, und zum anderen verschließen sich die definierten Durchlässe aufgrund ihrer Größe und ihres Randes bei Bedarf im eingesetzten Zustand zuverlässig.

Das Gefäßprothesensystem umfasst erfindungsgemäß eine Gefäßprothese zum Einführen in ein Blutgefäß, insbesondere ein Hauptblutgefäß, beispielsweise in die Aorta, eines menschlichen Körpers. Das Gefäßprothesensystem kann erfindungsgemäß weiterhin vorzugsweise mindestens eine Gefäßstütze aufweisen. Weiterhin kann das Gefäßprothesensystem eine Vorrichtung zum Zuführen von Mitteln in das Blut aufweisen. Weitere zum Einbringen der Gefäßprothese notwendigen Vorrichtungen, wie Führungsdraht, Ballonkatheter und dergleichen können Teil des Gefäßprothesensystems sein. Vorzugsweise werden aber nur die im menschlichen Körper verbleibenden Teile, insbesondere Gefäßprothese und Gefäßstütze als Teile des Gefäßprothesensystems bezeichnet.

Als Gefäßprothese wird erfindungsgemäß, wie auch allgemein im Stand der Technik, eine in der Regel hohlzylindrische Vorrichtung bezeichnet, die in der Regel (selbstexpandierende) Federelemente mit Formgedächtnis, die auch als Stents bezeichnet werden, zum Abstützen an der Gefäßwand aufweist. Die Gefäßprothese wird daher auch als Graft, Endograft oder Stentgraft bezeichnet. Die Gefäßprothese weist ein Gefäßprothesenmaterial auf, das beispielsweise Dacron oder ein anderes Stoffgewebe darstellt. Das Gefäßprothesenmaterial bildet die Mantelfläche der Gefäßprothese. In dem oder an dem Gefäßprothesenmaterial können Federelemente vorgesehen, bspw. angenäht, sein, die das Gefäßprothesenmaterial im eingebrachten Zustand der Gefäßprothese an die Gefäßwand drücken und die Gefäßprothese so in Position halten.

Als "Gefäßstütze" wird dabei vorliegend jede Struktur verstanden, die geeignet ist, ein Gefäß offenhaltend zu stützen und über die Gefäßprothese verankert zu werden. Gemäß einer bevorzugten Ausführungsform ist die Gefäßstütze ein gecoverter Stent.

Das in der Gefäßprothese erfindungsgemäß vorgesehene zumindest eine Loch stellt eine Verschlusseinrichtung zum Verschließen des Gefäßprothesenmaterials gegen Blutdurchdringung dar. Dabei wird als "Verschließen" des Materials der Gefäßprothese insbesondere die Einstellung einer Dichtigkeit gegen ungewollten Blutaustritt aus der Gefäßprothese und damit gegen ungewolltes Vorbeiströmen von Blut an der Gefäßprothese verstanden. Überraschenderweise wurde im Rahmen der vorliegenden Erfindung festgestellt, dass entgegen der normalen Erwartung die Einbringung/das Vorsehen eines oder mehrerer Löcher in das Material der Gefäßprothese zu einer gezielten Beeinflussung der Blutdurchlässigkeit genutzt werden kann.

Dabei wird vorliegend als "Loch" erfindungsgemäß eine Öffnung bezeichnet, die eine Größe aufweist, die größer als die Maschenweite des Materials der Gefäßprothese ist. Gleichzeitig ist das Loch erfindungsgemäß aber kleiner als der Durchmesser einer an der Gefäßprothese zu befestigenden Gefäßstütze im eingebrachten Zustand. Die Größe oder der Durchmesser x des Lochs bezeichnet die Abmessung des Lochs in einem Zustand in dem das Loch (noch) nicht geweitet ist. Das Loch stellt vorzugsweise eine runde Öffnung dar. Es ist aber auch möglich, dass das Loch eine ovale, rechteckige oder andere Form aufweist.

Vorzugsweise sind an der Gefäßprothese dabei mehrere Löcher in dem Lochungsbereich vorgesehen. Diese sind vorzugsweise zueinander benachbart in das Material der Gefäßprothese eingebracht. Der Bereich, in dem die Löcher vorgesehen sind, wird daher auch als Lochungsbereich bezeichnet. Der Lochungsbereich erstreckt sich vorzugsweise nur über einen Teil der Länge der Gefäßprothese, und weist einen Lochungsbereichlänge a auf. In Umfangsrichtung kann der Lochungsbereich sich über den gesamten Umfang b der Gefäßprothese erstrecken. Es versteht sich, dass alternativ der Lochungsbereich nur auf einer Hälfte, einem Viertel oder einem anderen Teilbereich des Umfangs b der Gefäßprothese vorgesehen sein kann.

Indem an der Gefäßprothese eine Verschlusseinrichtung in Form mindestens eines Lochs vorgesehen ist, wird zum einen erreicht, dass durch das Loch in dem Zustand der Gefäßprothese, in dem diese in das Hauptblutgefäß eingebracht ist, ein Führungsdraht auf einfache Weise durch das Material der Gefäßprothese, insbesondere durch eines der Löcher, geführt werden kann. Zum anderen wird dadurch, dass das Loch als Verschlusseinrichtung dient, ein ungewollter Blutaustritt aus der Gefäßprothese verhindert. Zudem können gemäß einer weiteren Ausführungsform vorzugsweise mehrere Löcher vorgesehen sein, wodurch die Anforderungen an die Positionierung der Gefäßprothese in dem Hauptblutgefäß gesenkt werden, da der Führungsdraht über ein beliebiges der vorgesehenen Löcher eingebracht werden kann. Das Vorsehen mehrerer Löcher ist aber nur bei der erfindungsgemäßen Gefäßprothese möglich, da Löcher, die nach dem Setzen einer Gefäßstütze in einem der Löcher unbenutzt sind, als Verschlusseinrichtung dienen und somit den Bereich um die Gefäßstütze abdichten. Zudem wird durch die vorliegende Erfindung eine von dem Gefäß unabhängige Orientierung der Gefäßstütze ermöglicht. Insbesondere kann bei einer Gefäßstütze, bei der sich der Lochungsbereich über den Umfang der Gefäßstütze erstreckt, die Gefäßstütze in einer beliebigen Orientierung um deren Längsachse in ein Gefäß eingebracht werden, an dem Gefäßabzweigungen vorliegen, da durch den Lochungsbereich die Gefäßstütze die Gefäßabzweigung zwar überdeckt, aber dennoch eine Perfusion gewährleistet.

Gemäß einer Ausführungsform kann das Gefäßprothesensystem mindestens eine Zuführvorrichtung zum Zuführen eines die Fließeigenschaften des Blutes beeinflussendes Mittels in den menschlichen Körper umfassen. Die Zuführvorrichtung stellt insbesondere eine Vorrichtung dar, über die das die Fließeigenschaften des Blutes beeinflussende Mittel dosiert werden kann, das heißt über die die Menge und die Dauer der Zuführung eingestellt werden können. Eine mögliche Ausführungsform der Zuführvorrichtung stellt somit eine Pumpe dar, bei der das Fördervolumen gesteuert werden kann. Die Zuführvorrichtung ist vorzugsweise ein zu der Gefäßprothese separates Teil des Gefäßprothesensystems. Die Zuführung in den Blutkreislauf erfolgt an einer geeigneten, das heißt für den behandelnden Arzt leicht zugänglichen Stelle beispielsweise über eine Kanüle, die in ein Blutgefäß im Arm eingebracht wird. Die Zuführung des die Fließeigenschaften des Blutes beeinflussenden Mittels erfolgt systemisch.

Das die Fließeigenschaften des Blutes beeinflussende Mittel kann ein gerinnungsbeeinflussendes Mittel sein oder umfassen. Vorzugsweise ist dieses Mittel ein gerinnungshemmendes Mittel, insbesondere Heparin. Das gerinnungshemmende Mittel kann auch als Thrombinhemmer, thrombozytenaggregationshemmendes oder antithrombotisches Mittel bezeichnet werden. Die durch das gerinnungshemmende Mittel bewirkte Antikoagulation führt dazu, dass das Blut nicht oder schwerer gerinnt. Hierdurch bleibt bei Zugabe eines solchen Mittels oder zumindest Aufrechterhaltung einer bestimmten Konzentration des Mittels die Fließfähigkeit des Blutes gegeben. Alternativ oder zusätzlich zu gerinnungshemmenden Mitteln können auch blutverdünnende Mittel verwendet werden, durch die die Viskosität des Blutes verringert wird. Schließlich kann als die Fließeigenschaften des Blutes beeinflussendes Mittel auch ein Mittel eingesetzt werden, das thrombosierende Eigenschaften aufweist oder ein Antagonisieren eines gerinnungshemmenden Mittels, wie beispielsweise Heparin, bewirkt. Ein solches Mittel zum Antagoniesieren, kann beispielsweise Protamin sein. Durch Zugabe von Protamin kann beispielsweise eine zuvor durch Zugabe von Heparin eingestellte Heparin-Konzentration im Blut gesenkt werden.

Durch Hemmung der Gerinnung des Blutes und/oder Verringerung der Viskosität kann bei der erfindungsgemäßen Gefäßprothese eine Blutzufuhr durch die Löcher in die abzweigenden Blutgefäße, die durch den Lochungsbereich abgedeckt sind, sichergestellt werden. Dies ist während der Dauer des Setzens der Gefäßstütze in dem abzweigenden Blutgefäß von Bedeutung, da in dieser Zeit eine ausreichende Durchblutung der Abzweigungsgefäße beziehungsweise Blutzufuhr zu den Abzweigungsgefäßen erforderlich ist. Indem durch die Zugabe oder Zuführung des Mittels die Fließeigenschaften des Blutes beeinflusst werden, kann die der aktuellen Situation angepasste Fließeigenschaft erzeugt werden. Insbesondere kann eine gewünschte Konzentration eines Mittels in dem Blut eingestellt werden.

Da zudem die Größe der Löcher so gewählt ist, dass diese als Verschlusseinrichtung dienen können, kann bei Herabsetzen oder vollständigem Einstellen der Zuführung des die Fließeigenschaften des Blutes beeinflussenden Mittels, insbesondere eines Gerinnungshemmers oder Blutverdünners, das Blut das Loch beziehungsweise die Löcher verschließen und dadurch einen weiteren Durchlass von Blut verhindern.

Gemäß einer Ausführungsform ist die Größe des Lochs daher so klein, dass unbehandeltes oder geringfügig mit einem die Fließeigenschaften des Blutes beeinflussenden Mittel behandeltes menschliches Blut das Loch nach einer bestimmten Durchströmzeit verschließt.

Der Einfachheit halber wird in dieser Beschreibung in der Regel auf ein Loch Bezug genommen. Es versteht sich aber, dass die Ausführungen - soweit anwendbar - bei den erfindungsgemäßen mehreren Löchern auf jedes der mehreren Löcher zutreffen. Erfindungsgemäß können bis 100, vorzugsweise bis 15 Löcher vorgesehen sein, vorzugsweise 2, 3, 4, 56, 7, 8, 9, 10, 11, 12, 13, 14, 15, 16, 17, 18, 19, 20, 25, 30, oder mehr Löcher, wobei vom Erfindungsgedanken auch von den angegebenen Bereichen umfasste, aber nicht explizit aufgeführte Zahlen mit umfasst sind.

Als unbehandeltes Blut wird in diesem Zusammenhang Blut bezeichnet, dem kein Mittel, das die Fließeigenschaften des Blutes beeinflusst, zugegeben ist oder wird. Geringfügig behandeltes Blut hingegen bezeichnet Blut, dem geringe Mengen eines solchen Mittels zugegeben wurden oder werden oder bei dem die Konzentration des Mittels im Blut gesenkt wurde. Der Zustand des Blutes ist der jeweils aktuelle Zustand. So kann unbehandeltes Blut zeitlich vorher mit dem Mittel behandelt worden sein beziehungsweise eine höhere Konzentration aufgewiesen haben.

Das Loch weist erfindungsgemäß einen Durchmesser im Bereich von 1,0 mm bis 1,3 mm auf. Besonders bevorzugt besitzt das Loch einen Durchmesser von 1,2 mm. Die Größe des Loches wird nach unten dadurch begrenzt, dass dieses mindestens dem Durchmesser eines Führungsdrahtes, der zum Setzen der Gefäßstütze durch das Loch geführt werden muss, entspricht.

Gemäß einer Ausführungsform ist die Größe des Lochs so groß, dass zumindest mit einem die Fließeigenschaften des Blutes beeinflussenden Mittel behandeltes Blut durch das Loch strömen kann. Die Größe des Lochs ist nach oben durch den Durchmesser der einzubringenden Gefäßstütze begrenzt. Die Größe des Lochs ist dabei erfindungsgemäß stets kleiner als der Durchmesser der einzubringenden Gefäßstütze. Zudem ist die Größe des Lochs nach oben durch die Maximalgrenze der Zugabe eines die Fließeigenschaften des Blutes beeinflussenden Mittels begrenzt. Beispielsweise bei gerinnungshemmenden Mitteln ist eine Maximalgrenze dadurch gegeben, oberhalb derer es zu Blutungen kommen kann, das heißt das Blut aus den Blutgefäßen austreten kann. Die Größe des Lochs wird daher so gewählt, dass bei einer zulässigen Menge und Dauer der Zugabe des Mittels ein Durchströmen von Blut durch das Loch gewährleistet ist.

Erfindungsgemäß weist das mindestens eine Loch ferner einen geschlossenen, also durchgehenden, umlaufenden Rand aus dem Material der Gefäßprothese auf. Als geschlossener (durchgehender) umlaufender Rand wird hierbei ein Rand bezeichnet, über den sich kein Material in Richtung des Loches über den Rand hinaus erstreckt. Insbesondere stehen keine Fadenenden über den Rand hinaus in das Loch hinein. Zudem ist der Rand fixiert, was bedeutet, dass der Rand die Form des Loches hält. Erfindungs- und definitionsgemäß wird damit das Loch bzw. der Rand des zumindest einen Lochs nicht durch Maschen, d.h. durch sich kreuzende Fäden gebildet; eine Masche hat definitionsgemäß keinen umlaufenden, geschlossenen bzw. durchgehenden Rand, sondern wird durch sich überkreuzende Fäden definiert, die an der Überkreuzung nicht fixiert sind. Somit unterscheiden sich das oder die erfindungsgemäßen Löcher von einem Zwischenraum zwischen Fäden, also Maschen, des Materials der Gefäßprothese. Wie gerade diskutiert, werden diese Zwischenräume zwar von Fäden des Materials umgeben, die Fäden sind aber nicht fixiert und es kann daher zu einem Verschieben der Fäden kommen, durch die sich auch die Form des Zwischenraums verändert.

Bei dem erfindungsgemäß vorgesehenen Rand hingegen kann auch bei dem erforderlichen Zusammenfalten der Gefäßprothese zum Einführen in das Hauptblutgefäß die Form des Loches beibehalten werden und dieses liegt dann an der Abzweigung von dem Hauptblutgefäß zum Durchführen eines Führungsdrahtes in der gewünschten Form vor.

Gemäß einer bevorzugten Ausführungsform wird der Rand des Loches beziehungsweise werden die Ränder der Löcher thermisch fixiert. Hierbei können entweder überstehende Fadenenden abgeschmolzen oder zueinander durch Aufdehnen des Materials der Gefäßprothese verschobene Fäden in einer relativen Position zueinander fixiert werden.

Erfindungsgemäß kann an der Gefäßprothese zumindest in dem Lochungsbereich zumindest ein Verstärkungsmittel an dem Material der Gefäßprothese vorgesehen sein.

Wie weiter oben ausgeführt, stellt der Lochungsbereich eine Art Verschlussvorrichtung der Gefäßprothese dar, da sich das/die im Lochungsbereich vorgesehene Loch/Löcher aufgrund ihrer Dimension und ihres Designs Blutfluss gegenüber verschließen können. Durch das Vorsehen von Verstärkungsmitteln kann ein Überdehnen des Materials der Gefäßprothese in dem Lochungsbereich beim Aufdehnen eines der Löcher zum Einführen einer Gefäßstütze und beim Setzen einer Gefäßstütze verhindert werden. Dadurch kann der Halt der Gefäßstütze an der Gefäßprothese weiter verbessert werden und das Austreten von Blut auch an dem Übergang zwischen Gefäßprothese und Gefäßstütze verhindert werden.

Dabei wird unter einem "Verstärkungsmittel" jede Struktur verstanden, die geeignet ist, in dem bzw. an dem Lochungsbereich angebracht zu werden, und diesem dadurch eine Stabilität verleiht, welche ein Aufdehnen des/der Lochs/Löcher verhindert.

Das Verstärkungsmittel ist gemäß einer Ausführungsform vorzugsweise in Abständen an dem Material der Gefäßprothese vorgesehen und kann bspw. bezüglich Längsachse der Gefäßprothese winklig vorgesehen sein.

Gemäß einer Ausführungsform stellt das Verstärkungsmittel eine Verstärkungsnaht dar. Vorzugsweise ist zwischen zwei Verstärkungsmitteln mindestens eines der Löcher in dem Material der Gefäßprothese vorgesehen. Zu diesem Zweck können die Verstärkungsnähte vorzugsweise gekreuzt vorgesehen sein, so dass zwischen den Nähten Rechtecke oder Karos entstehen. In jedem oder in einigen dieser Rechtecke oder Karos sind jeweils vorzugsweise mehrere Löcher in das Material der Gefäßprothese eingebracht.

Alternativ zu Verstärkungsnaht kann das Verstärkungsmittel auch eine Verstärkungsstruktur, beispielsweise eine Netzstruktur oder Gitterstruktur sein. Solche Strukturen können auch als stentähnliche Strukturen bezeichnet werden. Durch die Verwendung solcher Strukturen kann zum einen das Material der Gefäßprothese unterstützt werden und zum anderen kann auch die Gefäßprothese als solches stabilisiert werden. Die stentähnliche Struktur kann beispielsweise aus Metall, Kunststoff oder anderen bekannten Materialien für Medizinprodukte bestehen, die eine gewisse Flexibilität aufweisen, um ein Zusammenfalten der Gefäßprothese zum Einführen in das Gefäß zu ermöglichen. Das Verstärkungsmittel, das eine stentähnliche Struktur darstellt, kann beispielsweise an der Innenseite oder der Außenseite des Materials der Gefäßprothese vorgesehen sein. Es ist aber auch möglich, dass die stentähnliche Struktur in das Material der Gefäßprothese eingearbeitet ist, das heißt in diesem Material liegt.

Die Erfindung ist nicht auf die beschriebenen Ausführungsformen begrenzt. Beispielsweise kann in dem Lochungsbereich der Gefäßprothese eine größere Maschenweite des Materials der Gefäßprothese als in den weiteren Bereichen der Gefäßprothese, insbesondere dem Federbereich, vorgesehen sein. Hierdurch wird das Aufweiten zum Einführen und Setzen der Gefäßstütze weiter vereinfacht.

Alternativ zu einer größeren Maschenweite des Materials in dem Lochungsbereich oder zusätzlich dazu kann auch vorgesehen sein, dass in dem Lochungsbereich eine Bindungsart verwendet wird, die sich von der Bindungsart des weiteren Bereiches des Materials der Gefäßprothese, beispielsweise dem Federbereich unterscheidet. Zudem kann vorgesehen sein, dass der Lochungsbereich aus aneinander angrenzenden Teilbereichen unterschiedlicher Maschenweite und/oder unterschiedlicher Bindungsarten besteht. Als Bindungsarten werden unterschiedliche Webarten bezeichnet. Beispielsweise kann in dem Lochungsbereich oder in einem Teilbereich des Lochungsbereiches eine Webart verwendet werden, bei der der Schussfaden über mindestens zwei Kettfäden verläuft, bevor er unter den nächsten Kettfaden geführt wird. Insbesondere können Köperbindungen und/oder Atlasbindungen in dem Lochungsbereich oder in Teilbereichen des Lochungsbereiches verwendet werden.

Der Teil des Materials der Gefäßprothese, der außerhalb des Lochungsbereiches, beispielsweise im Federbereich, liegt, kann hingegen beispielsweise aus einem Material bestehen, bei dem die Fäden durch Leinwandbindung miteinander verbunden sind, das heißt bei dem der Schussfaden nur über einen Kettfaden verläuft, bevor er unter den nächsten Kettfaden geführt wird.

Sind unterschiedlichen Teilbereiche in dem Lochungsbereich mit unterschiedlichen Bindungsarten vorgesehen, kann insbesondere der Vorteil erzielt werden, dass das Aufdehnungsvermögen in dem Bereich eines Loches, durch das eine Gefäßstütze geführt und gehalten werden soll, durch die Bindungsart eines Teilbereiches gegeben sein kann, die Aufdehnung aber durch einen angrenzenden Teilbereich anderer Webart gehindert wird, das heißt ein Überdehnen des Materials der Gefäßprothese durch die Gefäßstütze verhindert werden kann. Zudem ist auch das Einbringen der Löcher in dem Lochungsbereich vereinfacht, da diese gezielt in Bereichen oder Teilbereichen vorgesehen werden können, in dem weniger Bindungspunkte vorliegen, beispielsweise in einem Teilbereich mit Atlasbindung. Hierdurch wird ein Aufdehnen zum Bilden des Loches vereinfacht. Gemäß einer Ausführungsform können beispielsweise viereckige Teilbereiche in dem Lochungsbereich gebildet sein, die ein Schachbrettmuster bilden, wobei die viereckigen Teilbereiche abwechselnd Atlas und Köper Teilbereiche darstellen können. Durch die Verwendung unterschiedlicher Bindungsarten oder Webarten, kann zum einen das Einbringen der Löcher in den Lochbereich erleichtert werden, durch angrenzende Teilbereiche andere Bindungsart oder Webart aber auch das Überdehnen des Loches beim Setzen der Gefäßstütze zuverlässig verhindert werden.

Gemäß einer bevorzugten Ausführungsform umfasst das Gefäßprothesensystem zusätzlich zu der Gefäßprothese mindestens eine Gefäßstütze. Die Gefäßstütze wird im Folgenden auch als Stent bezeichnet. Der Stent stellt hierbei einen gecoverten, das heißt mit einem Material an dem Umfang versehenen Stent dar.

Gemäß einer Ausführungsform des erfindungsgemäßen Gefäßprothesensystems umfasst die Gefäßstütze einen Stent, der an einem Ende durch einen zylindrischen Bereich gebildet und weist an dem gegenüberliegenden Ende einen erweiterten Bereich mit größerem Durchmesser auf. Das Ende mit dem erweiterten Bereich ist das Ende, das beim Setzten des Stents in die Gefäßprothese eingeführt wird. Durch das Vorsehen eines solchen erweiterten Bereiches kann eine zuverlässige Verankerung des Stents an bzw. in der Gefäßprothese gewährleistet werden. Da zudem die Außenseite des Stents an dem Rand des Loches in dem Material der Gefäßprothese anliegt, ist die Verbindung zwischen Stent und Gefäßprothese zusätzlich abgedichtet.

Der Durchmesser der Gefäßstütze in dem zylindrischen Bereich entspricht vorzugsweise dem Durchmesser eines der Löcher in einem gedehnten Zustand. Als gedehnter Zustand wird der Zustand des Loches bezeichnet, der durch mechanisches Expandieren des Durchmessers Lochs, bspw. über eine Ballon, erzeugt wird. Das Dehnen oder Aufweiten des Loches erfolgt an der Gefäßprothese in dem Zustand, in dem diese bereits in dem Hauptblutgefäß positioniert ist. Der zylindrische Bereich kann gleich dem Durchmesser eines der Löcher im gedehnten Zustand sein. Vorzugsweise ist der zylindrische Bereich aber geringfügig größer als das Loch im gedehnten Zustand. Eine solche Bemessung des zylindrischen Bereiches der Gefäßstütze, die auch als Oversizing bezeichnet wird, führt zu einem besseren Halt der Gefäßstütze an dem Loch. Der Durchmesser des erweiterten Bereichs der Gefäßstütze ist vorzugsweise größer als der Durchmesser des Lochs in dem gedehnten Zustand. Durch diese Dimensionierung kann der erweiterte Bereich als Anker dienen und es wird möglich die Gefäßstütze von außen durch das aufgeweitete Loch in die Gefäßprothese einzuführen und zu setzen. Zum Einführen der Gefäßstütze in das aufgeweitete Loch liegt diese vorzugsweise zunächst in einem zusammenfalteten Zustand, beispielsweise in einem Katheter, vor und expandiert sich durch Zurückziehen des Katheters von selber. Zu diesem Zweck weist die Gefäßstütze mindestens ein Federelement auf, das in radialer Richtung nach außen vorgespannt ist.

Gemäß einer weiteren Ausführungsform ist vorgesehen, wenn die Gefäßprothese und/oder die Gefäßstütze in ihrer Längsrichtung hintereinander angeordnete Ringe aus mäanderförmig umlaufenden Stützen aufweist, wobei die Ringe nicht untereinander, sondern lediglich über das Gefäßprothesenmaterial miteinander verbunden sind.

Bei dieser Ausführungsform sind also einzelne Stentringe, die abwechselnd in proximale oder distale Richtung weisen über, nur bzw. lediglich indirekt über das Gefäßprothesenmaterial miteinander verbunden, und nicht direkt miteinander.

Gemäß einer Ausführungsform ist dabei vorgesehen, wenn insbesondere Am proximalen und/oder distalen Ende Stentringe vorgesehen sind; diese können mit Gefäßprothesenmaterial bedeckt bzw. an oder in diesem angebracht, also gecovert, oder ungecovert sein.

Gemäß einem weiteren Aspekt betrifft die vorliegende Erfindung ein Verfahren zur Herstellung einer Gefäßprothese eines erfindungsgemäßen Gefäßprothesensystems. Das Verfahren, das im Folgenden als Herstellungsverfahren bezeichnet wird, ist dadurch gekennzeichnet, dass nach oder während der Einbringung des mindestens einen Lochs in das Material der Gefäßprothese eine thermische Behandlung des Materials der Gefäßprothese zumindest in dem Bereich des mindestens einen Lochs erfolgt. Als thermische Behandlung wird insbesondere ein Erwärmen oder Erhitzen des Materials bezeichnet. Hierbei kann das Material der Gefäßprothese zumindest bereichsweise schmelzen. Durch thermische Behandlung des Materials kann insbesondere der Rand des Loches fixiert werden und ein Überstand von Fäden oder dergleichen kann beseitigt werden.

Das Einbringen der Löcher in das Material der Gefäßprothese erfolgt vor dem Einführen der Gefäßprothese in das Hauptblutgefäß.

Gemäß einer Ausführungsform des erfindungsgemäßen Herstellungsverfahrens wird das mindestens eine Loch mittels eines Werkzeuges eingebracht. Das Werkzeug kann ein Stanzwerkzeug sein. Vorzugsweise wird aber ein zerstörungsfreies Herstellungsverfahren benutzt, bei dem das Werkzeug beispielsweise eine Nadel oder Kanüle umfasst. Diese kann in das Material der Gefäßprothese eingebracht werden und hierbei die Fäden des Materials der Gefäßprothese verschieben.

Gemäß einer Ausführungsform bei der Verwendung eines Werkzeugs, das zumindest eine Nadel umfasst, werden die Nadel oder die Nadeln vor dem Einbringen in das Material der Gefäßprothese erwärmt. Alternativ kann die Nadel oder können die Nadeln auch nach dem Einbringen erwärmt werden. Statt Nadel können bei dem erfindungsgemäßen Verfahren auch beispielsweise Hohlnadeln oder Kanülen verwendet werden.

Alternativ zu einem Kontaktwerkzeug, wie beispielsweise einem Stanzwerkzeug oder einem Werkzeug mit Nadeln oder Kanülen kann das mindestens eine Loch auch durch kontaktlose Werkzeuge, insbesondere durch ein Laserwerkzeug, eingebracht werden. Hierbei wird das Loch mittels eines Laserstrahls, der auf das Material der Gefäßprothese gerichtet wird, eingebracht. Auch bei dieser Art der Einbringung wird vorzugsweise der Rand des Loches aufgrund einer durch den Laser erzeugten erhöhten Temperatur verschweißt oder versiegelt. Auch bei dieser Art der Einbringung mittels Laser wird somit während der Einbringung des mindestens einen Lochs in das Material der Gefäßprothese eine thermische Behandlung des Materials der Gefäßprothese zumindest in dem Bereich des mindestens einen Lochs durchgeführt.

Hierin offenbart ist auch ein Verfahren zum Einbringen einer Gefäßprothese eines Gefäßprothesensystems in ein Hauptblutgefäß eines Menschen. Das Verfahren, das im Folgenden auch als Einbringungsverfahren bezeichnet wird, ist dadurch gekennzeichnet, dass die Gefäßprothese in ein Hauptblutgefäß eines menschlichen Körpers eingeführt wird und ein die Fließeigenschaften des Blutes beeinflussendes Mittel in den Blutkreislauf zugeführt wird.

Die Reihenfolge des Einführens der Gefäßprothese und des Zuführens des Mittels zur Beeinflussung der Fließeigenschaften des Blutes ist nicht auf eine bestimmte Reihenfolge begrenzt. Somit kann vor dem Einführen der Gefäßprothese bereits mit der Zufuhr des Mittels erfolgen. Alternativ kann zunächst die Gefäßprothese eingeführt werden und anschließend die Zufuhr des Mittels begonnen werden. Schließlich kann auch während des Einführens der Gefäßprothese mit der Zufuhr begonnen werden.

Wesentlich ist aber, dass es sich um eine Zuführung, das heißt eine gezielte Zugabe, die nach Zeit und Menge variiert werden kann, handelt. Das reine Vorsehen eines Mittels zur Beeinflussung der Fließeigenschaften des Blutes an der Gefäßprothese stellt somit kein Zuführen im Sinne der Offenbarung dar, da hierbei ein gezieltes Variieren der Menge über die Zeit nicht möglich ist und auch die Menge nicht gezielt variiert, insbesondere verringert werden kann.

Z.B. kann das die Fließeigenschaften des Blutes beeinflussende Mittel über eine Dauer und in einer Menge zugegeben, dass das mindestens eine Loch der Gefäßprothese für das Durchströmen von Blut offen ist. Bei einem gerinnungshemmenden Mittel wird hierzu insbesondere über eine gewisse Zeit die Zugabemenge des Mittels oder zumindest die Konzentration des Mittels in dem Blut gesteigert. Das Gerinnen von Blut, das zu einem Zusetzen des Loches oder der Löcher führen kann, wird hierbei unterbunden oder zumindest gehemmt.

Das Einführen der Gefäßprothese erfolgt bspw. mit Hilfe eines Katheters, in dem die Gefäßprothese aufgenommen ist. Die Gefäßprothese wird an der gewünschten Position freigesetzt, das heißt aus dem Katheter entlassen. Die Position, in der die Gefäßprothese freigesetzt wird, liegt vorzugsweise im Bereich einer Abzweigung eines oder mehrerer Abzweigungsgefäße von dem Hauptblutgefäß. Die Gefäßprothese wird so positioniert, dass der Lochungsbereich der Gefäßprothese die Abzweigung oder die Abzweigungen überdeckt. Indem Mittel zur Beeinflussung der Fließeigenschaften des Blutes zugeführt wird, kann in diesem Zustand sichergestellt werden, dass Blut durch die Löcher des Lochungsbereiches der Gefäßprothese weiterhin in die Abzweigungsgefäße gelangen kann, das heißt dass in dem Lochungsbereich weiterhin eine Perfusion gewährleistet wird.

Ein Führungsdraht kann durch das Hauptblutgefäß oder durch ein Abzweigungsgefäß durch den Lochungsbereich der Gefäßprothese durch eines der Löcher geführt werden und mittels eines Ballons wird das Loch aufgeweitet.

Nach dem Aufweiten eines der Löcher der Gefäßprothese wird bspw. eine Gefäßstütze über das Hauptblutgefäß oder ein Abzweigungsgefäß in das aufgeweitete Loch der Gefäßprothese zumindest bereichsweise eingeführt und freigesetzt. Als Freisetzen wird hierbei das Aufweiten der Gefäßstütze verstanden, dass bei einer selbstexpandierenden Gefäßstütze beispielsweise durch Zurückziehen eines Katheters, in dem die Gefäßstütze aufgenommen ist, erfolgen kann.

Nach dem Setzen der Gefäßstütze durch Verringerung der Zugabe des die Fließeigenschaften des Blutes beeinflussenden Mittels wird mindestens eines der Löcher der Gefäßprothese verschlossen. Hierbei werden nur die Löcher verschlossen, in denen keine Gefäßstütze vorgesehen wurde. Das Loch, das geweitet wurde und in das die Gefäßstütze eingeführt wurde, wird durch die Gefäßstütze offen gehalten und im aufgeweiteten Zustand gehalten. Da der Durchmesser der Gefäßstütze vorzugsweise größer als der Durchmesser des Loches im aufgeweiteten Zustand ist, wird die Gefäßstütze durch das Anliegen an dem Rand des Loches gehalten. Wird zusätzlich eine Gefäßstütze mit einem erweiterten Bereich an einem Ende verwendet, kann dieser erweiterte Bereich zusätzlich zur Halterung der Gefäßstütze an der Gefäßprothese dienen.

An den Löchern, an denen keine Gefäßstütze eingeführt wurde, kommt es aufgrund der Verringerung der Zuführung des Mittels zur Beeinflussung der Fließeigenschaften des Blutes zu einem Verschließen der Löcher. Wird ein gerinnungshemmendes Mittel verwendet, tritt bei Verringerung der Zuführung des Mittels eine Gerinnung auf, die zum Zusetzen der Löcher führt. Ist das Mittel ein Blutverdünner wird bei Verringerung der Zuführung des Mittels die Viskosität des Blutes verringert bis dieses nicht mehr durch die Löcher durchtreten kann.

Die Verringerung der Zuführung des die Fließeigenschaften des Blutes beeinflussenden Mittels erfolgt nach dem Freisetzen der Gefäßstütze.

Statt der Verringerung der Zuführung des die Fließeigenschaften des Blutes beeinflussenden Mittels kann auch die Zugabe eines anderen die Fließeigenschaften des Blutes beeinflussenden Mittels erfolgen. Dieses andere Mittel wirkt bspw. der Wirkweise eines zuvor zugegebenen Mittels entgegen und/oder verringert die Konzentration des zuvor zugegebenen Mittels im Blut. Beispielsweise kann zunächst Heparin zugegeben werden und zur Verringerung der Herparin-Konzentration im Blut anschließend Protamin zugeführt werden.

Das die Fließeigenschaften des Blutes beeinflussende Mittel wird vorzugsweise in der Dauer und Menge zugegeben, um einen Verschluss von mindestens einem der Löcher gezielt zu beeinflussen. Hierbei wird die Zugabe oder Zuführung beispielsweise nach der folgenden Formel bestimmt:

$$V = L_K \left( f B_{V0}^{\,10.000} \right)$$

Der Verschluss ist hiernach eine Funktion der Lochgröße (Lk), die konstant ist, und der Zuführung des Mittels zur Beeinflussung der Fließeigenschaften des Blutes (Bv). Die Zuführung des Mittels zur Beeinflussung der Fließeigenschaften des Blutes kann in den Grenzen 0 bis 10.000 variiert werden. Diese Grenzen können als Werte in IE/ml angegeben werden und beziehen sich insbesondere auf das Mittel Heparin. Die Dosierung von Heparin wird in IE (Internationale Einheiten) angegeben. 1.000 IE vermögen 1l Schafs- oder Rinderblut bei 37°C eine Stunde lang ungerinnbar zu machen.

Beim oder nach dem Einführen der Gefäßprothese in das Hauptblutgefäß kann bspw. Heparin in einer Menge von mehr als 0,75 IE/ml zugeführt werden. Dies bedeutet, dass bei einer normalen Blutmenge eines Menschen von 6 Liter mindestens 4500 IE Herparin zugeführt beziehungsweise im Körper gehalten werden müssen. In diesem Zustand können, insbesondere bei einer Lochgröße in dem offenbarten Bereich , die Löcher offen gehalten werden, das heißt Blut kann in die Abzweigungsgefäße strömen. Nach dem Setzen der Gefäßstütze sollen die nicht durch die Gefäßstütze besetzten Löcher des Lochungsbereiches verschlossen werden. Zu diesem Zweck kann die Zugabe von Heparin oder die Konzentration des Heparin im Blut auf einen Wert unter 0,75 IE/ml gesenkt werden. Es hat sich gezeigt, dass bei dieser Menge an Heparin der Verschluss der Löcher im Lochbereich durch Koagulation des Blutes beginnt.

Der kritische Wert der sogenannten Activated Clotting Time (ACT) des Blutes liegt bspw. im Bereich von 150 bis 250 Sekunden, bspw. bei 200 Sekunden. Solange ein ACT Wert erzielt wird, der über dem kritischen Wert liegt, werden die Löcher des Lochungsbereiches offen gehalten. Sinkt der ACT Wert unter den kritischen Wert, beginnen der Verschluss der Löcher und damit das Abdichten der Gefäßprothese.

Die Erfindung wird im Folgenden erneut unter Bezugnahme auf die beiliegenden Zeichnungen genauer beschrieben. Es zeigen

Figur 1:    eine schematische Darstellung einer Ausführungsform einer Gefäßprothese des erfindungsgemäßen Gefäßprothesensystems;

Figur 2:    eine schematische Detaildarstellung eines Teils des Lochungsbereiches der Ausführungsform der Gefäßprothese nach Figur 1;

Figur 3:    eine schematische Detaildarstellung des Teils des Lochungsbereiches der Ausführungsform der Gefäßprothese nach Figur 2 im aufgeweiteten Zustand des Lochs;

Figur 4:    eine schematische Darstellung einer Ausführungsform der Einbringung eines Lochs in das Material einer Gefäßprothese;

Figur 5:    eine schematische Darstellung einer weiteren Ausführungsform der Einbringung eines Lochs in das Material einer Gefäßprothese;

Figur 6:    eine schematische Darstellung einer Verwendung einer Ausführungsform der erfindungsgemäßen Gefäßprothese im Bereich der Nierenarterie;

Figur 7:    eine schematische Darstellung einer Verwendung einer Ausführungsform der erfindungsgemäßen Gefäßprothese im Bereich der Communis Arterie;

Figur 8:    eine schematische Darstellung einer Verwendung einer Ausführungsform der erfindungsgemäßen Gefäßprothese im Bereich Carotis Arterie und Subclavia Arterie;

Figur 9:    eine schematische Darstellung der Verwendung einer Ausführungsform der erfindungsgemäßen Gefäßprothese im Bereich der Carotis Arterie und Subclavia Arterie nach Figur 8 beim Einbringen einer Gefäßstütze; und

Figur 10:   eine schematische Darstellung einer Ausführungsform einer Gefäßstütze des erfindungsgemäßen Gefäßprothesensystems.

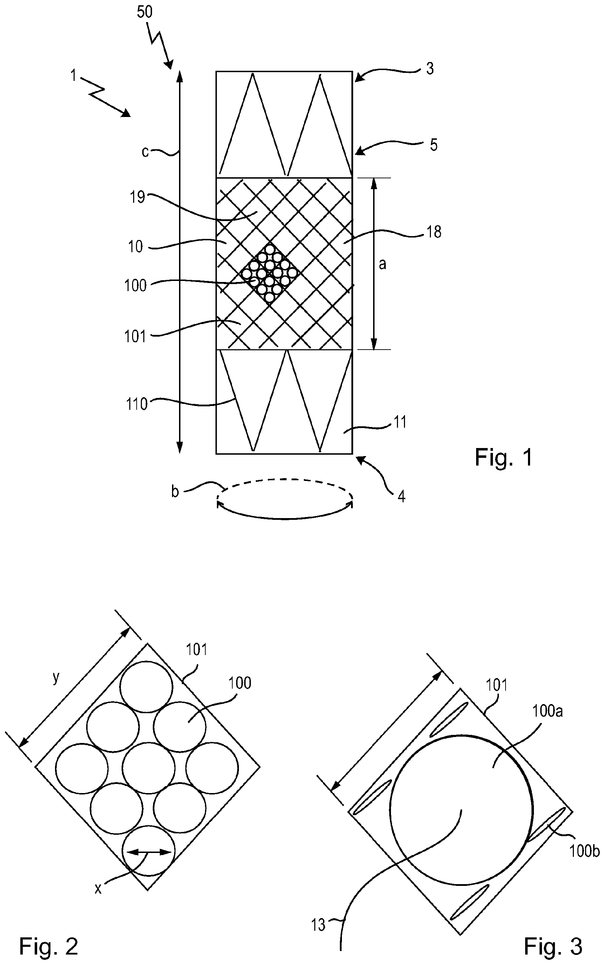

In Figur 1 ist eine schematische Darstellung einer Ausführungsform einer Gefäßprothese 1 des erfindungsgemäßen Gefäßprothesensystems 50 gezeigt. Die Gefäßprothese 1 weist in der dargestellten Ausführungsform einen länglichen hohlzylindrischen Grundkörper 5 mit einem proximalen Ende 3 und einem distalen Ende 4 auf. An den Enden 3, 4 der Gefäßprothese 1 sind Federbereiche 11 vorgesehen, in denen Federelemente 110 in oder an dem Material 12 der Gefäßprothese 1 befestigt sind. Zwischen den Federbereichen 11 ist ein Lochungsbereich 10 vorgesehen, der eine Länge a aufweist. In diesem Lochungsbereich 10 sind in das Material 12 der Gefäßprothese 1 Löcher 100 eingebracht, die Verschlusseinrichtungen gemäß der Erfindung darstellen.

Weiterhin sind in dem in Fig. 1 gezeigten Beispiel der erfindungsgemä-ßen Gefäßprothese im Lochungsbereich 10 Verstärkungsmittel 101 in Form von sich kreuzenden Verstärkungsnähten vorgesehen. Die Verstärkungsnähte sind so angeordnet, dass zwischen diesen Karos gebildet sind. Alternativ zu den gezeigten Verstärkungsnähten kann beispielsweise aber auch eine stentähnliche Verstärkungsstruktur (nicht gezeigt) an dem Material 12 der Gefäßprothese 1 vorgesehen sein oder in dieses Material 12 eingearbeitet sein. Zudem kann zumindest ein Teilbereich in dem Lochungsbereich 10 oder der gesamte Lochungsbereich 10 eine Bindungsart aufweisen, die sich von der Bindungsart der Federbereiche 11 unterscheidet. Sind Teilbereiche gebildet, können diese beispielsweise viereckig sein, so dass die Verteilung der Teilbereiche im Lochungsbereich 10 ein Schachbrettmuster ergeben.

Die Verteilung der Löcher 100 in den in Figur 1 gezeigten Karos ist in Figur 2 genauer gezeigt. Wie sich aus der Figur 2 ergibt, werden in einem Bereich, der eine Längs- und Breitenabmessung Y aufweist und daher vorzugsweise rechteckig ist, Löcher 100 eingebracht, die einen Durchmesser x aufweisen, der kleiner ist als Y. Y stellt den senkrechten Abstand zwischen zwei Verstärkungsnähten dar. Der Lochungsbereich 10, über den die Löcher in der Länge der Gefäßprothese 1 vorgesehen sind, erstreckt sich über eine Länge a, die größer als die Flächenhalbierende des Karos mit der Länge und Breite Y ist.

Wird, wie in Figur 3 gezeigt, ein Loch 100a durch Einführen eines Führungsdrahtes 13 und Expandieren eines daran vorgesehenen Ballons (nicht gezeigt), geweitet, wird die Ausdehnung des Loches 100a durch die Verstärkungsmittel 101 begrenzt. Die weiteren Löcher 100b, die mit dem erweiterten Loch in einem Karo liegen, werden beim Aufdehnen des Lochs 100a zusammengedrückt, das heißt verkleinert, und bilden schlitzartige Formen, wie in Fig. 3 gezeigt.

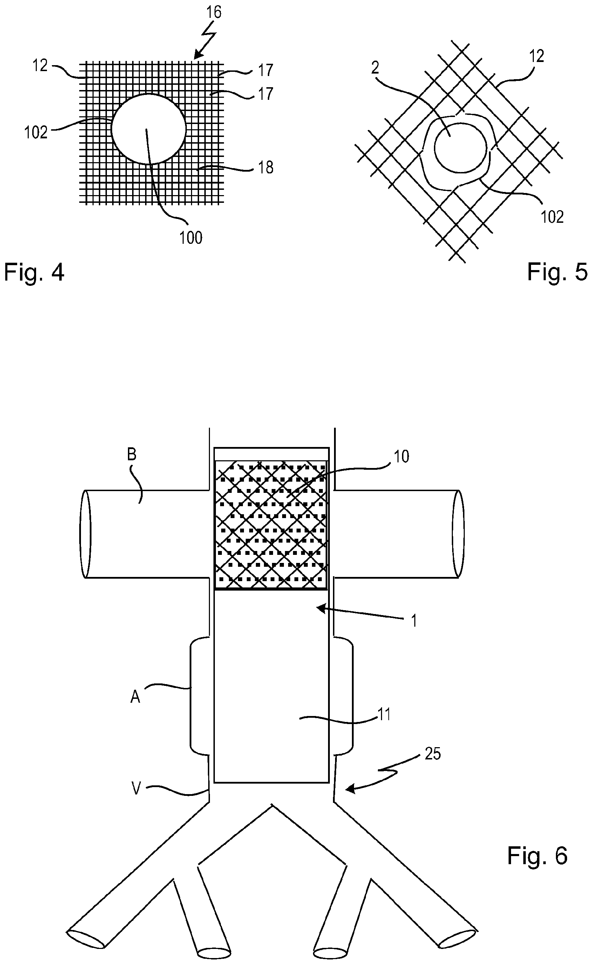

In Figur 4 ist eine schematische Darstellung einer Ausführungsform der Einbringung eines Lochs 100 in das Material 12 einer erfindungsgemäßen Gefäßprothese 1 gezeigt. Hierbei wird das Loch 100 beispielsweise durch Stanzen oder Ausschneiden des Materials 12 der Gefäßprothese 1 erzeugt. Das Material 12 der Gefäßprothese 1 ist hier schematisch durch gewobene Fäden 17 gezeigt. Der Umfang des Lochs 100 wird durch einen Rand 102 gebildet. In der in Figur 4 gezeigten Ausführungsform werden hierzu die Fadenenden, die an dem Umfang des Lochs 100 vorliegen, miteinander verschweißt oder verschmolzen. Zu diesem Zweck kann beispielsweise eine erwärmte Nadel oder Kanüle (nicht gezeigt) in das Loch 100 eingebracht werden, und zwar für eine Dauer die ausreicht, um die Fadenenden miteinander zu verschweißen.

In Figur 5 ist eine schematische Darstellung einer weiteren Ausführungsform der Einbringung eines Lochs 100 in das Material 12 einer Gefäßprothese 1 gezeigt. Bei dieser Ausführungsform wird das Loch 100 zerstörungsfrei in das Material 12 der Gefäßprothese 1 eingebracht. Insbesondere wird eine Nadel 2 in das Material 12 eingeführt und verdrängt dabei die Fäden 17 des Materials 12 nach außen. Durch Erwärmen der Nadel 2 kann die so gebildete relative Position der Fäden 17 zueinander fixiert werden und damit der Rand 102 des Lochs 100 gebildet werden. Alternativ kann das Loch 100 auch durch Laserbehandlung in das Material 12 der Gefäßprothese 1 eingebracht werden.

In Figur 6 ist eine schematische Darstellung einer beispielhaften Überstentung der Nierenarterie 25 mit einer Ausführungsform der erfindungemäßen Gefäßprothese 1 gezeigt. An dem Hauptblutgefäß V der Nierenarterie 25 ist in Figur 6 schematisch ein Defekt in Form eines Aneurysma A gezeigt. Um dieses zu entlasten und ein Versagen des Hauptblutgefäßes V zu verhindern, wird die Gefäßprothese 1 in das Hauptblutgefäß V eingebracht. Die Gefäßprothese 1 wird dabei so positioniert, dass der in dem Ausführungsbeispiel gezeigten gecoverten Federbereich 11 das Aneurysma A überdeckt. In dem Bereich, in dem der Federbereich 11 an dem Aneurysma A anliegt, müssen allerdings nicht zwangsweise Federelemente vorgesehen sein. In Figur 6 sind der besseren Erkennbarkeit halber keine Federelemente gezeigt. Der Lochungsbereich 10 liegt an den Abzweigungsgefäßen B, die auch als Abgangsgefäße bezeichnet werden und in Figur 6 die Nierenarterien darstellen.

Es versteht sich, dass in dem in Fig. 6 gezeigten Beispiel mit der Nierenarterie nur ein beispielhaftes Gefäß dargestellt ist. Das erfindungsgemäße Gefäßprothesensystem kann in jedes Gefäß eingeführt werden, mit dem Ziel, von dem Gefäß abzweigende Seitengefäße sicher mit Blut zu versorgen, wenn eine Gefäßprothese in das Gefäß eingeführt wird und die Seitengefäße zu blockieren droht.

Vor, während oder nach dem Positionieren der Gefäßprothese 1 wird ein die Fließeigenschaften des Blutes beeinflussenden Mittel systemisch in den Blutkreislauf eingebracht. Die Löcher 100 des Lochungsbereiches 10 werden dadurch offen gehalten.

Durch die so positionierte Gefäßprothese 1 kann ein Führungsdraht (nicht gezeigt) geführt werden und in dem Lochungsbereich 10 durch eines der darin vorgesehenen Löcher 100 in eines der Abzweigungsgefäße B geführt werden. Anschließend kann das Loch 100, durch das der Führungsdraht geführt wurde, mechanisch aufgeweitet werden, bspw. mittels eines Ballons (nicht gezeigt). Anschließend kann eine Gefäßstütze (in Fig. 6 nicht gezeigt) durch das aufgeweitete Loch 100 in das Abzweigungsgefäß B gebracht und dort freigesetzt werden. Nach dem Setzen der Gefäßstütze wird die Zufuhr des Mittels zur Beeinflussung der Fließeigenschaften des Blutes verringert oder die Konzentration des Mittels in dem Blut auf andere Weise beispielsweise durch Zuführung eines anderen Mittels verringert. Hierdurch verschließen sich die weiteren Löcher 100, in denen keine Gefäßstütze vorgesehen ist, im Lochungsbereich 10 und der Weg des Blutes ist durch die Gefäßprothese 1 sowie die Gefäßstütze vorgegeben.

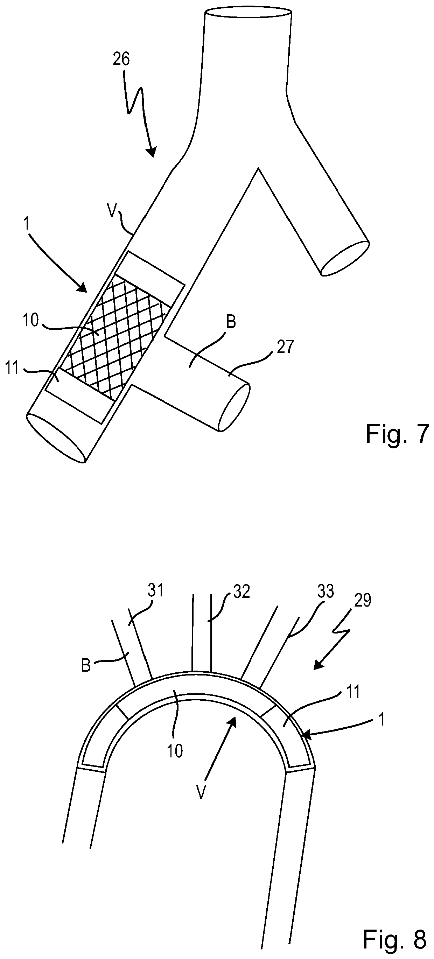

In Figur 7 ist eine schematische Darstellung einer Überstentung im Bereich der Communis Arterie 26 mit einer Ausführungsform der erfindungemäßen Gefäßprothese 1 gezeigt. Auch in dieser Figur ist lediglich die Gefäßprothese 1 in der zum Setzen der Gefäßstütze notwendigen Position gezeigt, und nicht auch die Gefäßstütze. Die Gefäßprothese 1 ist in die Carotis Communis Arterie 26, bzw. in das Hauptgefäß V, eingefügt und der Lochungsbereich 10 überdeckt die Abzweigung zu der Carotis Externa Arterie 27, bzw. des Abgangsgefäßes B. Die Löcher 100 im Lochungsbereich 10 sind in dieser Ansicht nicht gezeigt.

In Figur 8 ist eine schematische Darstellung einer Überstentung im Bereich der vom Aortenbogen 29 abgehenden Carotis Arterie und Subclavia Arterie mit einer Ausführungsform der erfindungemäßen Gefäßprothese 1 gezeigt. Hierbei ist die Gefäßprothese 1 in den Aortenbogen 29 eingebracht und der Lochungsbereich 10 überdeckt die rechte und linke Carotis Communis 31, 32 sowie die Subclavia Arterie 33. In dieser Ausführungsform erstreckt sich somit der Lochungsbereich 10 der Gefäßprothese 1 über (alle) drei Abzweigungen.

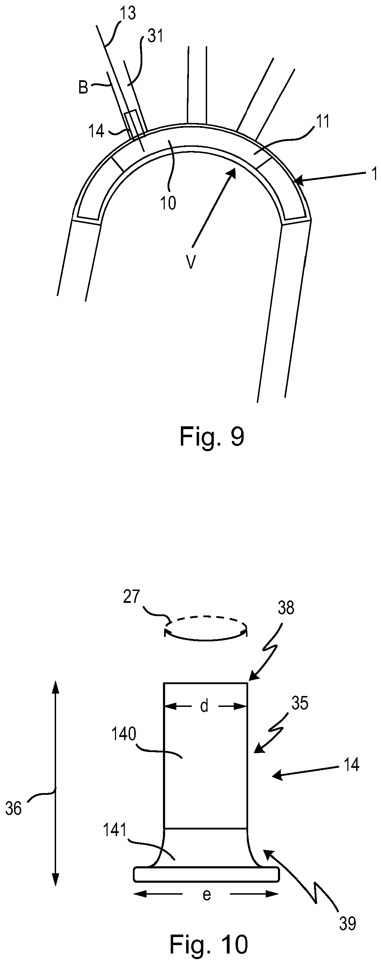

Wie sich aus Figur 9 ergibt, die eine schematische Darstellung der Überstentung der Carotis Arterien 31, 32 und Subclavia Arterie 33 nach Figur 8 beim Einbringen einer Gefäßstütze 14 in die rechte Carotis Communis zeigt, kann mit der vorliegenden Erfindung die Gefäßstütze 14 von außen an die Gefäßprothese 1 gebracht werden.

Eine Ausführungsform der Gefäßstütze 14, bspw. ein Stent, ist schematisch in Figur 10 gezeigt. Die Gefäßstütze 14, die einen hohlzylindrischen Gefäßstützengrundkörper 35, eine Längsachse 36, sowie einen Umfang 37 umfasst, weist ein erstes Gefäßstützenende 38 mit einem ersten Durchmesser d und ein zweites Gefäßstützenende 39 mit einem zweiten Durchmesser e auf, der vorliegend auch als erweiterter Bereich 141 bezeichnet wird. Diese Gefäßstütze 14 kann von außen in die Gefäßprothese 1 durch ein vorher aufgeweitetes Loch 100 in dem Lochungsbereich 10 eingeführt werden und wird nach dem Freisetzen der Gefäßstütze 14 durch den erweiterten Bereich 141 an bzw. in der Gefäßprothese 1 gehalten.

Auch bei den Ausführungsbeispielen nach Figuren 7, 8 und 9 wird vor, während oder nach dem Positionieren der Gefäßprothese 1 ein die Fließeigenschaften des Blutes beeinflussendes Mittel systemisch in den Blutkreislauf eingebracht und überwacht. Die Löcher 100 des Lochungsbereiches 10 werden dadurch offen gehalten. Nach dem Setzen der Gefäßstütze 14 wird die Zufuhr des Mittels zur Beeinflussung der Fließeigenschaften des Blutes verringert oder die Konzentration des Mittels in dem Blut auf andere Weise beispielsweise durch Zuführung eines anderen Mittels verringert. Hierdurch verschließen sich die weiteren Löcher 100 im Lochungsbereich 10 und der Weg des Blutes ist durch die Gefäßprothese 1 und die Gefäßstütze 14 vorgegeben.

Bei der erfindungsgemäßen Gefäßprothese 1 erfolgt somit ein Verschluss der eingebrachten Löcher 100 durch Beeinflussung der Fließeigenschaften, insbesondere des Gerinnungsverhaltens des Blutes. Wie weiter oben diskutiert, verschließen sich die Löcher 100 nach Einstellung der Zuführung des Mittels zur Beeinflussung der Fließeigenschaften.

Abzweigungsgefäße beziehungsweise Abgangsgefäße B werden bei der erfindungsgemäßen Gefäßprothese 1 zwar überdeckt. Da allerdings in diesem Bereich der Lochungsbereich mit den Löchern vorgesehen ist, ist die Blutströmung zu oder von dem abzweigenden Gefäß B nicht behindert. Zu diesem Zweck wird die Gerinnungsneigung des Blutes vorzugsweise beeinflusst. Alternativ oder zusätzlich wird das Blut verdünnt. Hierzu können bekannte Gerinnungshemmer oder Blutverdünnungsmittel zugegeben werden. Vorzugsweise wird Heparin verwendet. In diesem Zustand, in dem die Löcher durch die Blutverdünnung offen gehalten sind, kann ein Draht mit einem Ballon, insbesondere Dilatationsballon durch eines der Löcher 100 in der Gefäßprothese 1 hindurch geführt werden, da der Lochungsbereich 10 der Gefäßprothese 1 in dem Bereich der Abzweigung platziert wurde. Der Draht mit dem Ballon wird dazu verwendet, das Loch 100, durch das dieser hindurchgeführt wurde, aufzuweiten. Durch das so aufgeweitete Loch 100 wird dann eine Gefäßstütze 14, insbesondere ein gecoverter Stent, eingesetzt. Der Stent erstreckt sich hierbei von dem aufgeweiteten Loch in die Richtung des abzweigenden Gefäßes B, und liegt somit in dem abzweigenden Gefäß B.

Somit kann mit der vorliegenden Erfindung ein intraoperatives Fenestrieren vorgenommen werden.

Gemäß einer bevorzugten Ausführungsform sind in dem Lochungsbereich 10 Nähte oder eine stentähnliche Verstärkungsstruktur 101 vorgesehen, die ein Weiten der Löcher in dem Material 12 der Gefäßprothese 1, das auch als Graftmaterial bezeichnet werden kann und insbesondere Drakon oder einem anderen Tuchmaterial ist, begrenzen.

In dem Lochungsbereich 10 kann die Gefäßprothese 1 grober geflochten oder gewebt sein. Hierdurch wird das Weiten der Löcher 100 mittels des Ballons vereinfacht. Zusätzlich oder alternativ können auch unterschiedliche Bindungsarten in dem Lochungsbereich 10 vorliegen, beispielsweise Köper und Atlas.

Die Erfinder haben erkannt, dass eine Beziehung zwischen der Lochgröße/Lochdurchmesser x, der Blutverdünnung und dem sich einstellenden Verschlusses des Loches 100 besteht. Insbesondere ist der Verschluss eine Funktion der konstanten Lochgröße/Lochdurchmessers x und dem Verlauf der Blutverdünnung.

Bei dem offenbarten Einbringungsverfahren und mit der erfindungsgemäßen Gefäßprothese 1 kann vorzugsweise ein Stent, insbesondere ein gecoverter Stent als Gefäßstütze 14 verwendet werden, der eine Form aufweist, bei der ein Ende des Stents im eingebrachten Zustand einen größeren Durchmesser aufweist als der restliche Stent. Insbesondere kann der Stent eine Trompetenform aufweisen. Der Bereich 141 der Gefäßstütze/des Stents, der den größeren Durchmesser aufweist, liegt im eingebrachten Zustand an der Innenseite der Gefäßprothese 1 an, während der restliche Stent sich in dem abzweigenden Gefäß B befindet.

Zum Einführen des Stents/der Gefäßstütze 14 wird daher wie folgt vorgegangen. Zunächst wird der Stent/die Gefäßstütze 14 in das Gefäß, beispielsweise in die Aorta im Bereich des Aortenbogens 29 eingebracht. Hierbei überdeckt der Lochungsbereich 10, in dem die Löcher 100 in das Material 12 der Gefäßprothese 1 eingebracht sind, die Abgangsgefäße B, insbesondere die Carotis 31, 32 und Subclavia 33. In diesem Zustand kann ein Draht gegen den in die Abgangsgefäße gerichteten Blutfluss eingeführt werden. Dabei wird der Draht durch eines der Löcher 100 geführt und mittels des an dem Draht vorgesehenen oder geführten Ballons wird das Loch 100 gedehnt. Anschließend kann über den Draht ein Stent/eine Gefäßstütze 14 eingeführt werden, bis dessen/deren distales Ende in der Gefäßprothese 1 liegt. In diesem Zustand kann der Stent/die Gefäßstütze 14 freigesetzt werden. Durch das Freisetzen dehnt sich der Stent/die Gefäßstütze 14 aus und wird dadurch an bzw. in der Gefäßprothese 1 befestigt. Durch die bevorzugte Form des Stents/der Gefäßstütze 14, insbesondere die Trompetenform, kann der Halt des Stents/der Gefäßstütze 14 an der Gefäßprothese 1 noch verbessert werden. Der Bereich mit größerem Durchmesser 141 wird über das Loch 100 hinaus bis ins Innere der Gefäßprothese 1 geführt. Beim Freisetzen des Stents/der Gefäßstütze 14 weitet dieser/diese sich aus und der Bereich des größeren Durchmessers 141 fungiert als Anker.

Nach dem Setzen des Stents/der Gefäßstütze 14 ist es erfindungsgemäß vorgesehen, dass der Lochungsbereich 10 verschlossen wird. Dies erfolgt erfindungsgemäß durch Beeinflussen der Fließeigenschaften des Blutes, insbesondere durch Einstellen der Blutverdünnung oder Blutgerinnung.

Zur Beeinflussung der Fließeigenschaften des Blutes und damit zum gezielten Verschließen von Löchern 100 in einem Bereich der Gefäßprothese 1 kann ein Blutverdünnungsmittel oder Gerinnungshemmer in unterschiedlichen Dosierungen zugeben werden beziehungsweise die Dosierung des Blutverdünnungsmittels oder Gerinnungshemmers variiert werden oder die Konzentration des Mittels in dem Blut auf andere Weise beispielsweise durch Zuführung eines anderen Mittels verringert werden.

Der Gerinnungshemmer kann beispielsweise Heparin sein. In einem Bereich von 7.000 bis 10.000 Einheiten (IE/ml) hat sich gezeigt, dass die Löcher 100 in der Gefäßprothese 1 zuverlässig offen gehalten werden können und somit ein Blutstau beziehungsweise das Abschneiden der Blutzufuhr zu bestimmten Blutgefäßen während des Einbringens der Gefäßprothese 1 und dem Setzen eines oder mehrerer Stents/Gefäßstützen 14 an der Gefäßprothese 1 verhindert werden kann.

Somit kann eine Kombination aus einem geeigneten Lochdurchmesser x und dem Blutzustand gewählt werden, der gezielt zum Verschließen der Löcher 100 führt. Der Verschluss der Löcher 100 ist nach dem Setzen des oder der Stents/Gefäßstützen 14 erforderlich.

## Patentansprüche

1. Gefäßprothesensystem (50), umfassend zumindest eine Gefäßprothese (1) zum Einführen in ein Blutgefäß eines menschlichen Körpers, wobei die Gefäßprothese (1) ein proximales Ende (3), ein distales Ende (4), sowie einen sich zwischen dem proximalen (3) und dem distalen (4) Ende länglichen hohlzylindrischen Grundkörper (5) mit einer Längsachse c und einem Umfang b aufweist, welcher ein Gefäßprothesenmaterial (12) zur Ausbildung eines umfänglich im Wesentlichen geschlossenen Mantels (19) aufweist, wobei das Gefäßprothesenmaterial (12) eine Gewebestruktur (18) aus miteinander verwobenen Fäden (17) und mit einer Maschendichte (16) besitzt, **dadurch gekennzeichnet, dass** die Gefäßprothese (1) im Gefäßprothesenmaterial (12) zumindest einen, zwischen dem proximalen (3) und dem distalen Ende (4) gelegenen Lochungsbereich (10) mit mehreren Löchern (100) aufweist, die einen Durchmesser x aufweisen, der ausgewählt ist aus einem Bereich von 1,0 mm bis 1,3 mm, und wobei die mehreren Löcher (100) einen geschlossenen umlaufenden Rand (102) aufweisen, über welchen Rand (102) sich kein Gefäßprothesenmaterial (12) in Richtung der Löcher (100) über den Rand (102) hinaus erstreckt.

2. Gefäßprothesensystem (50) nach Anspruch 1, **dadurch gekennzeichnet, dass** die Löcher (100) einen Durchmesser x von 1,2 mm aufweisen.

3. Gefäßprothesensystem (50) nach einem der vorstehenden Ansprüche, **dadurch gekennzeichnet, dass** der geschlossene umlaufende Rand (102) der mehreren Löcher (100) aus Gefäßprothesenmaterial (12) besteht, wobei der Rand (102) vorzugsweise thermisch fixiert ist.

4. Gefäßprothesensystem (50) nach einem der vorstehenden Ansprüche, **dadurch gekennzeichnet, dass** sich der Lochungsbereich (10) bezüglich der Längsachse der Gefäßprothese (1) über nur einen Teil der Gefäßprothese (1) erstreckt.

5. Gefäßprothesensystem (50) nach einem der vorstehenden Ansprüche, **dadurch gekennzeichnet, dass** der Lochungsbereich (10) bezüglich des Umfangs b der Gefäßprothese (1) derart dimensioniert ist, dass er sich über zumindest einen Teil des Umfangs b oder über den gesamten Umfang b erstreckt.

6. Gefäßprothesensystem (50) nach einem der vorstehenden Ansprüche, **dadurch gekennzeichnet, dass** im Lochungsbereich (10) ferner zumindest ein Verstärkungsmittel (101) am Gefäßprothesenmaterial (12) vorgesehen sind.

7. Gefäßprothesensystem (50) nach Anspruch 6, **dadurch gekennzeichnet, dass** das Verstärkungsmittel (101) ausgewählt ist aus einer Verstärkungsnaht oder einer Verstärkungsstruktur.

8. Gefäßprothesensystem (50) nach einem der Ansprüche 6 oder 7, **dadurch gekennzeichnet, dass** zwischen zwei Verstärkungsmitteln (101) zumindest ein Loch (100) im Gefäßprothesenmaterial (12) vorgesehen ist.

9. Gefäßprothesensystem (50) nach einem der Ansprüche 1 bis 8, **dadurch gekennzeichnet, dass** es ferner eine Gefäßstütze (14) mit einer Längsachse (36), einem Umfang (37), und einem hohlzylindrischen Gefäßstützengrundkörper (35) umfasst, welche Gefäßstütze (14) ferner ein erstes Gefäßstützenende (38) mit einem ersten Durchmesser d und ein zweites Gefäßstützenende (39) mit einem zweiten Durchmesser e aufweist, und welche Gefäßstütze (14) über das zweite Gefäßstützenende (39) in ein Loch (100) der mehreren Löcher (100) im Lochungsbereich (10) einbringbar ist.

10. Gefäßprothesensystem (50) nach Anspruch 9, **dadurch gekennzeichnet, dass** das zweite Gefäßstützenende (39) der Gefäßstütze (14) einen größeren Durchmesser e aufweist als das erste Gefäßstützenende (38) der Gefäßstütze (14).

11. Gefäßprothesensystem (50) nach einem der vorstehenden Ansprüche, **dadurch gekennzeichnet, dass** die Gefäßprothese (1) und/oder die Gefäßstütze (14) in ihrer Längsrichtung (c; 36) hintereinander angeordnete Ringe aus mäanderförmig umlaufenden Stützen aufweist, wobei die Ringe nicht untereinander, sondern lediglich über das Gefäßprothesenmaterial (12) miteinander verbunden sind.

12. Gefäßprothesensystem (50) nach einem der vorstehenden Ansprüche, **dadurch gekennzeichnet, dass** es ferner ein Zuführsystem zum Zuführen von zumindest einem die Fließeigenschaften von Blut beeinflussenden Mittels in den menschlichen Körper umfasst.

13. Verfahren zur Herstellung eines Gefäßprothesensystems (50) nach einem der Ansprüche 1 bis 12, **dadurch gekennzeichnet**, es den Schritt der Einbringung mehrerer Löcher mit einem Durchmesser x, der ausgewählt ist aus einem Bereich von 1,0 mm bis 1,3 mm (100) im Gefäßprothesenmaterial (12) aufweist, wobei nach oder während der Einbringung der mehreren Löcher (100) eine thermische Behandlung des Gefäßprothesenmaterials (12) in dem Bereich der mehreren Löcher (100) erfolgt.

14. Verfahren nach Anspruch 13, **dadurch gekennzeichnet, dass** die mehreren Löcher (100) mittels eines Laserwerkzeuges oder eines Werkzeuges eingebracht wird, das mindestens eine Nadel umfasst.

## Claims

1. A vessel prosthesis system (50) comprising at least one vessel prosthesis (1) for introduction into a blood vessel of a human body, wherein the vessel prosthesis (1) comprises a proximal end (3), a distal end (4), an oblong hollow cylindrical main body (5) extending in between the proximal end (3) and the distal end (4) having a longitudinal axis c and a circumference b, which main body (5) comprises a vessel prosthesis material (12) for forming a circumferentially substantially closed cover (19), wherein the vessel prosthesis material (12) comprises a fabric structure (18) from interwoven threads (17) and having a mesh density (16), **characterised in that** the vessel prosthesis (1) comprises, within the vessel prosthesis material (12), at least one perforation area (10) located in between the proximal end (3) and the distal end (4) with a plurality of perforations (100) comprising a diameter x selected from a range of 1.0 mm to 1.3 mm and wherein the plurality of perforations (100) comprise a closed circumferential rim (102), over which rim (102) no vessel prosthesis material (12) extends in the direction of the perforations (100) beyond the rim (102).

2. The vessel prosthesis system (50) of claim 1, **characterized in that** the perforations (100) comprise a diameter x of 1.2 mm.

3. The vessel prosthesis system (50) of any one of the preceding claims, **characterized in that** the closed circumferential rim (102) of the plurality of perforations (100) consists of vessel prosthesis material (12), wherein the rim (102) is preferably thermally fixed.

4. The vessel prosthesis system (50) of any one of the preceding claims, **characterized in that** the perforation area (10) extends with respect to the longitudinal axis of the vessel prosthesis (1) only over a part of the vessel prosthesis (1).

5. The vessel prosthesis system (50) of any one of the preceding claims, **characterized in that** the perforation area (10) is dimensioned with respect to the circumference b of the vessel prosthesis (1) such that it extends over at least a part of the circumference b or over the total circumference b.

6. The vessel prosthesis system (50) of any one of the preceding claims, **characterized in that** the in the perforation area (10) further at least one reinforcement means (101) are provided at the vessel prosthesis material (12).

7. The vessel prosthesis system (50) of claim 6, **characterized in that** the reinforcement means (101) is selected from a reinforcement suture or a reinforcement structure.

8. The vessel prosthesis system (50) of any one of claims 6 or 7, **characterized in that** in between of two reinforcement means (101) at least one perforation (100) is provided in the vessel prosthesis material (12).

9. The vessel prosthesis system (50) of any one of claims 1 to 8, **characterized in that** it further comprises a vessel support (14) with a longitudinal axis (36), a circumference (37), and a hollow cylindrical vessel support main body (35), which vessel support (14) further comprises a first vessel support end (38) having a first diameter d and a second vessel support end (39) having a second diameter e, and which vessel support (14) is introducible via the second vessel support end (39) into a perforation (100) of the plurality of perforations (100) in the perforation area (10).

10. The vessel prosthesis system (50) of claim 9, **characterized in that** the second vessel support end (39) of the vessel support (14) comprises a larger diameter e than the first vessel support end (38) of the vessel support (14).

11. The vessel prosthesis system (50) of any one of the preceding claims, **characterized in that** the vessel prosthesis (1) and/or the vessel support (14) comprises in its longitudinal direction (c; 36) successively arranged rings from meandering circumferential supports, wherein the rings are not connected with each other but only via the vessel prosthesis material (12).

12. The vessel prosthesis system (50) of any one of the preceding claims, **characterized in that** it further comprises an introduction system for introduction of at least one agent influencing the flow properties of blood into the human body.

13. A method for production of a vessel prosthesis system (50) of any one of claims 1 to 12, **characterized in that** it comprises the step of putting in, in the prosthesis material (12), a plurality of perforations (100) having a diameter x selected from a range of 1.0 mm to 1.3 mm (100), wherein after or during the putting in of the plurality of perforations (100) a thermal treatment of the vessel prosthesis material (12) is performed in the area of the plurality of perforations (100).

14. The method of claim 13, **characterized in that** the plurality of perforations (100) is put in by means of a laser tool or a tool that comprises at least one needle.

**Revendications**

1. Système à prothèse vasculaire (50), comprenant au moins une prothèse vasculaire (1) destinée à être insérée dans un vaisseau sanguin d'un corps humain, dans lequel la prothèse vasculaire (1) présente une extrémité proximale (3), une extrémité distale (4), ainsi qu'un corps de base (5) cylindrique creux s'étendant entre l'extrémité proximale (3) et l'extrémité distale (4) et comportant un axe longitudinal c et une circonférence b, lequel corps de base présente un matériau de prothèse vasculaire (12) pour la formation d'une gaine (19) essentiellement fermée sur la circonférence, dans lequel le matériau de prothèse vasculaire (12) possède une structure tissée (18) constituée de fils (17) entrelacés les uns avec les autres et comportant un maillage (16), **caractérisé en ce que** la prothèse vasculaire (1) présente, dans le matériau de prothèse vasculaire (12), au moins une zone perforée (10) située entre l'extrémité proximale (3) et l'extrémité distale (4) et comportant plusieurs trous (100) qui présentent un diamètre x qui est choisi dans une plage allant de 1,0 mm à 1,3 mm, et dans lequel les trous (100) présentent un bord (102) circonférentiel fermé, bord (102) au-delà duquel aucun matériau de prothèse vasculaire (12) ne s'étend en direction des trous (100) au-delà du bord (102).

2. Système à prothèse vasculaire (50) selon la revendication 1,
**caractérisé en ce que** les trous (100) présentent un diamètre x de 1,2 mm.

3. Système à prothèse vasculaire (50) selon l'une des revendications précédentes, **caractérisé en ce que** le bord (102) circonférentiel fermé des trous (100) est constitué de matériau de prothèse vasculaire (12), dans lequel le bord (102) est de préférence fixé thermiquement.

4. Système à prothèse vasculaire (50) selon l'une des revendications précédentes, **caractérisé en ce que** la zone perforée (10) s'étend sur une partie seulement de la prothèse vasculaire (1) par rapport à l'axe longitudinal de la prothèse vasculaire (1).

**5.** Système à prothèse vasculaire (50) selon l'une des revendications précédentes, **caractérisé en ce que** la zone perforée (10) est dimensionnée par rapport à la circonférence b de la prothèse vasculaire (1) de telle sorte qu'elle s'étend sur au moins une partie de la circonférence b ou sur la totalité de la circonférence b.

**6.** Système à prothèse vasculaire (50) selon l'une des revendications précédentes, **caractérisé en ce que,** dans la zone perforée (10), au moins un moyen de renforcement (101) est en outre prévu sur le matériau de prothèse vasculaire (12).

**7.** Système à prothèse vasculaire (50) selon la revendication 6,
**caractérisé en ce que** le moyen de renforcement (101) est choisi parmi une couture de renforcement ou une structure de renforcement.

**8.** Système à prothèse vasculaire (50) selon l'une des revendications 6 ou 7, **caractérisé en ce qu'**au moins un trou (100) est prévu entre deux moyens de renforcement (101) dans le matériau de prothèse vasculaire (12).

**9.** Système à prothèse vasculaire (50) selon l'une des revendications 1 à 8, **caractérisé en ce qu'**il comprend en outre un support vasculaire (14) comportant un axe longitudinal (36), une circonférence (37), et un corps de base de support vasculaire (35) cylindrique creux, lequel support vasculaire (14) présente en outre une première extrémité de support vasculaire (38) comportant un premier diamètre d et une seconde extrémité de support vasculaire (39) comportant un second diamètre e, et lequel support vasculaire (14) peut être introduit par l'intermédiaire de la seconde extrémité de support vasculaire (39) dans un trou (100) parmi les trous (100) dans la zone perforée (10).

**10.** Système à prothèse vasculaire (50) selon la revendication 9,
**caractérisé en ce que** la seconde extrémité de support vasculaire (39) du support vasculaire (14) présente un diamètre e supérieur à celui de la première extrémité de support vasculaire (38) du support vasculaire (14).

**11.** Système à prothèse vasculaire (50) selon l'une des revendications précédentes, **caractérisé en ce que** la prothèse vasculaire (1) et/ou le support vasculaire (14) présentent dans leur direction longitudinale (c ; 36) des anneaux disposés les uns derrière les autres et constitués de supports circonférentiels en forme de méandres, dans lequel les anneaux ne sont pas reliés entre eux, mais uniquement par l'intermédiaire du matériau de prothèse vasculaire (12).

**12.** Système à prothèse vasculaire (50) selon l'une des revendications précédentes, **caractérisé en ce qu'**il comprend en outre un système d'admission pour l'admission d'au moins un agent affectant les propriétés d'écoulement du sang dans le corps humain.

**13.** Procédé pour la fabrication d'un système à prothèse vasculaire (50) selon l'une des revendications 1 à 12, **caractérisé en ce qu'**il présente l'étape d'introduction de plusieurs trous comportant un diamètre x qui est choisi dans une plage allant de 1,0 mm à 1,3 mm (100) dans le matériau de prothèse vasculaire (12), dans lequel, après ou pendant l'introduction des trous (100), un traitement thermique du matériau de prothèse vasculaire (12) est effectué dans la zone des trous (100).

**14.** Procédé selon la revendication 13, **caractérisé en ce que** les trous (100) sont introduits au moyen d'un outil laser ou d'un outil comprenant au moins une aiguille.

Fig. 1

Fig. 2

Fig. 3

Fig. 4

Fig. 5

Fig. 6

Fig. 7

Fig. 8

EP 3 169 274 B1

Fig. 9

Fig. 10

18

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102012103987 A1
- US 20130296998 A1
- US 20120046728 A1